# EUROPEAN PATENT SPECIFICATION

(11) **EP 1 324 760 B1**
(45) Date of publication and mention of the grant of the patent: **20.06.2007**
(21) Application number: 01971000.3
(22) Date of filing: 14.09.2001
(51) Int. Cl.: A61K 31/221, A61K 9/48, A61K 9/20, A23L 1/305, C07C 67/00

(54) **CREATINE ESTER PRONUTRIENT COMPOUNDS AND FORMULATIONS**
CREATINESTER-PRONUTRIENT-VERBINDUNGEN UND FORMULIERUNGEN
COMPOSES ET FORMULATIONS DE SUPPLEMENTS D'ESTER DE CREATINE

(30) Priority: 14.09.2000 US 232969 P
(43) Date of publication of application: 09.07.2003
(60) Divisional application: 06075444.7
(73) Proprietor: Board of Regents of the University of Nebraska, Lincoln Nebraska 68583 (US)
(72) Inventor: VENNERSTROM, Jonathan, L., Omaha, NE 68124 (US); MILLER, Donald, W., Omaha, NE 68144 (US)
(74) Representative: McNeeney, Stephen Phillip
(86) International application number: PCT/US2001/028788
(87) International publication number: WO 2002/022135

(56) References cited:
- US-A- 5 576 316
- US-A- 5 773 473
- US-A- 5 994 581
- US-A- 6 093 848
- US-A- 6 117 872
- US-A- 6 136 339

## Description

The present invention generally relates to the field of creatine, and particularly to creatine ester pronutrient compounds and formulations.

### BACKGROUND OF THE INVENTION

Creatine is an endogenous nutrient produced naturally by the liver in most vertebrates. The uses of creatine are many, including use as a supplement to increase muscle mass and enhance muscle performance as well as in emerging applications in the treatment of neuromuscular disorders.

Typically, creatine is taken up into muscle cells by specific receptors and converted to phosphocreatine by creatine kinase. Muscle cells, including skeletal muscle and the heart muscle, function by utilizing cellular energy released from the conversion of adenosine triphosphate (ATP) to adenosine diphosphate (ADP). The amount of phosphocreatine in the muscle cell determines the amount of time it will take for the muscle to recover from activity and regenerate adenosine triphosphate (ATP). Phosphocreatine is a rapidly accessible source of phosphate required for regeneration of adenosine triphosphate (ATP) and sustained use of the muscle.

For example, energy used to expand and contract muscles is supplied from adenosine triphosphate (ATP). Adenosine triphosphate (ATP) is metabolized in the muscle by cleaving a phosphate radical to release energy needed to contract the muscle. Adenosine diphosphate (ADP) is formed as a byproduct of this metabolism. The most common sources of adenosine biphosphate (ATP) are from glycogen and creatine phosphate. Creatine phosphide is favored as a ready source of phosphate because it is able to resynthesize adenosine triphosphate (ATP) at a greater rate than is typically achieved utilizing glycogen. Therefore, increasing the amount of creatine in the muscle increases the muscle stores of phosphocreatine and has been proven to increase muscle performance and increase muscle mass.

US 6, 136, 339 discloses food supplements containing a combination of lipoic acid or a derivative thereof and creatine or a derivative thereof. The supplements preferably further comprise dextrose

However, creatine itself is poorly soluble in an aqueous solution. Further, creatine is not well absorbed from the gastrointestinal (GI) tract, which has been estimated to have a 1 to 14 percent absorption rate. Thus, current products require large amounts of creatine to be administered to be effective, typically 5 grams or more. Additionally, side effects such as bloating, gastrointestinal (GI) distress, diarrhea, and the like are encountered with these high dosages.

Therefore, it would be desirable to provide an improved approach for enhancing absorption of creatine.

### SUMMARY OF THE INVENTION

According to the present invention, there is provided a non-therapeutic method of providing creatine to an animal according to claim 1, a food supplement according to claim 20, and a method of producing a creatine pronutrient according to claim 35.

Accordingly, the present invention is directed to creatine ester pronutrients and formulations. In a first aspect of the present invention, a non-therapeutic method for providing creatine to an animal includes receiving a creatine ester by the animal. The creatine ester is suitable for being modified by the animal to form creatine.

In a second aspect of the present invention, a food supplement includes a creatine ester suitable for being modified by an animal to form creatine. In a third aspect of the present invention, a non-therapeutic method for providing creatine to an animal includes receiving an ester derivative of creatine by the animal. The ester derivative of creatine is suitable for acting as a pronutrient in an animal.

In a fourth aspect of the present invention, a composition of matter includes: 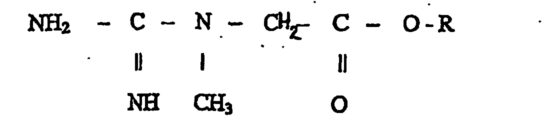 wherein R represents an ester.

In a fifth aspect of the present invention, a method of producing a creatine pronutrient includes reacting a hydrated form of creatine with an alcohol in an acidic environment wherein a product is formed including a creatine ester pronutrient.

It is to be understood that both the forgoing general description and the following detailed description are exemplary and explanatory only and are not restrictive of the invention as claimed. The accompanying drawings, which are incorporated in and constitute a part of the specification, illustrate an embodiment of the invention and together with the general description, serve to explain the principles of the invention.

### BRIEF DESCRIPTION OF THE DRAWINGS

The numerous advantages of the present invention may be better understood by those skilled in the art by reference to the accompanying figures in which:
FIG. 1A is an illustration depicting conversion of creatine to creatinine;
FIG. 1B is a depiction of an exemplary embodiment of the present invention wherein the processing of creatine monohydrate versus a creatine ester by the body is shown;
FIG. 1C is a flow diagram illustrating an exemplary embodiment of the present invention wherein a pronutrient derivative of creatine is created through the modification of an acid moiety by ester bond attachment;
FIG. 1D is an illustration of an embodiment of the present invention in which a graph depicting solubility and partition coefficients of creatine ethyl ester versus creatine monohydrate are shown;
FIGS. 2A, 2B, 2C, 2D, 2E, 2F, 2G, 2H, 2I, 2J, 2K, 2L, 2M and 2N are illustrations of exemplary compounds of the present invention;
FIG. 3 is an illustration depicting an exemplary embodiment of the present invention wherein a creatine ethyl ester compound is produced by solvating creatine monohydrate in dry ethyl alcohol in an acidic atmosphere;
FIG. 4 is an illustration of an embodiment of the present invention wherein additional methods and processes are shown for the production of a creatine ester; and
FIG 5 is an illustration depicting an exemplary embodiment of the present invention wherein a creatine benzyl ester compound is produced by solvating anhydrous creatine in dry benzyl alcohol in an acidic atmosphere.

### DETAILED DESCRIPTION OF THE INVENTION

Reference will now be made in detail to the presently preferred embodiments of the invention, examples of which are illustrated in the accompanying drawings.

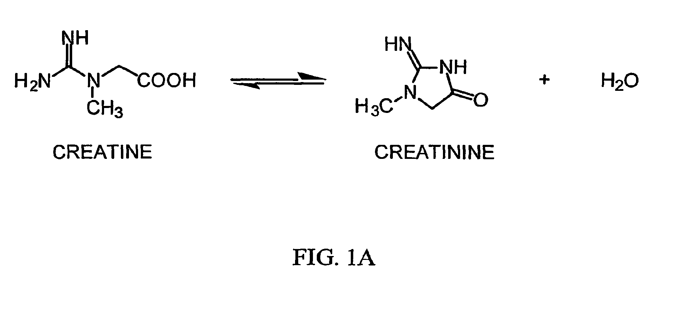

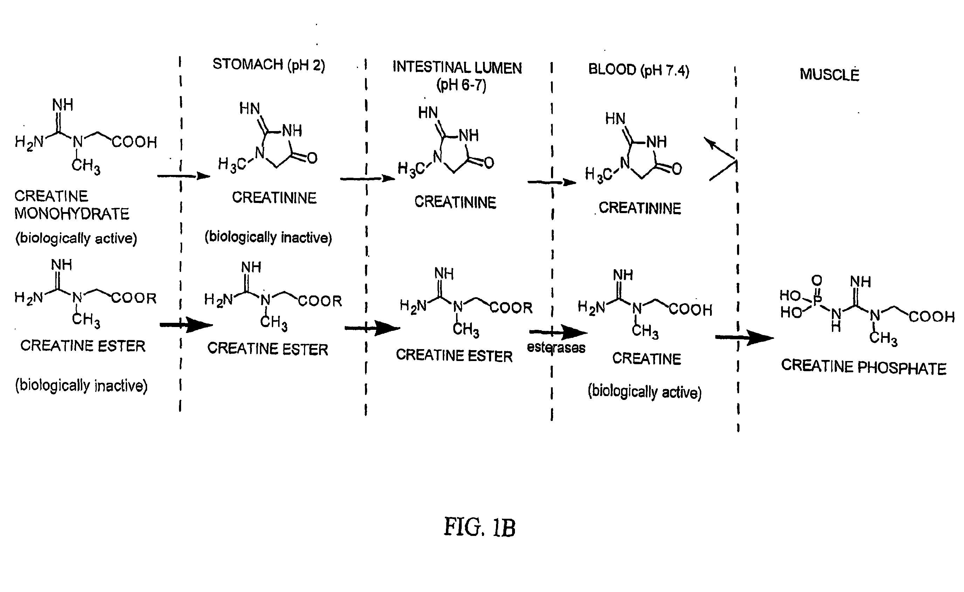

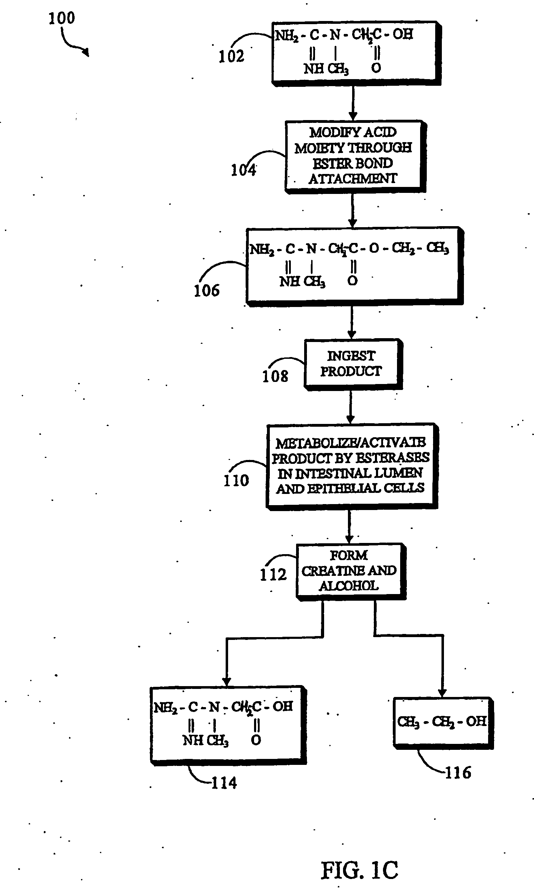

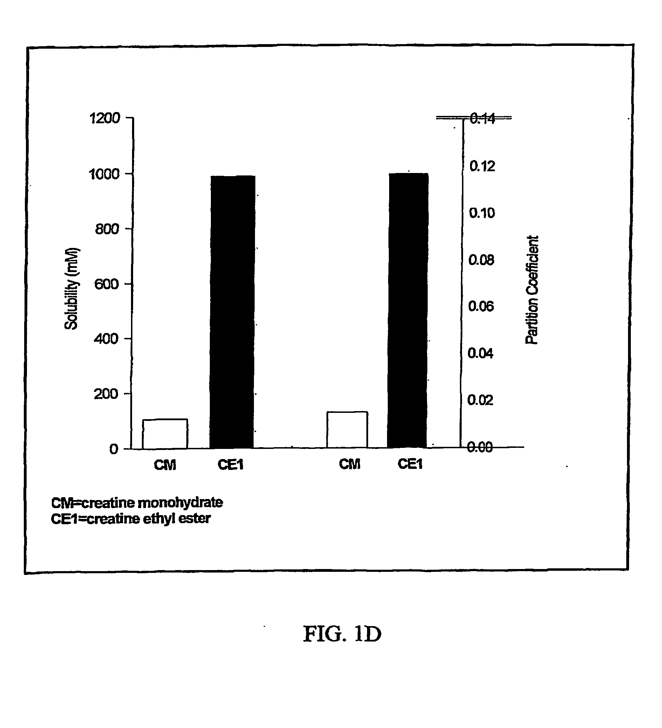

Referring generally now to FIGS. 1 through 5, exemplary embodiments of the present invention are shown. Creatine, N-aminoiminomethyl-N-methylglycine, is an endogenous nutrient which may be produced in the liver and kidneys. Typically, creatine is produced by the transfer of the guanidine moiety of arginine to glycine, which is then methylated to give creatine. Creatine may be represented by the following formula:

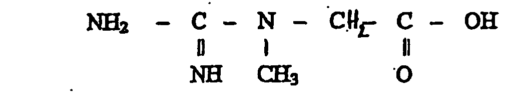

Creatine phosphate is formed in the body and may be represented by the following formula:

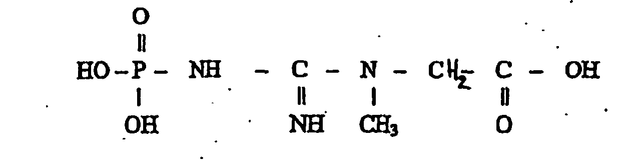

Creatine is converted to creatine phosphate by the creatine kinase enzyme. The creatine phosphate transfers its phosphate to adenosine diphosphate (ADP) to accomplish the regeneration of adenosine triphosphate (ATP). Adenosine triphosphate (ATP) may then be utilized by the muscles as a source of energy. Thus, by providing a formulation and method for enhanced absorption of creatine, the muscle levels of phosphocreatine will be elevated. As a result of this, muscle mass and performance may be increased, thereby permitting a variety of therapeutic applications.

Studies in the laboratory have shown that the aqueous solubility and partition coefficient of creatine monohydrate are 15.6 ± 2.1 mg/mL and 0.015 ± 0.007, respectively. The low oral bioavailability of creatine may derive not only from its low lipophilicity and concomitant poor membrane permeability, but also from rapid conversion to creatinine in the acidic condition of the stomach, and shown in FIG. 1A.

At a gastric pH range of 1-2, the equilibrium between creatine and creatinine shifts to the right such that the creatinine/creatine ratio may be greater than or equal to 30. See Bdgar, G.; Shiver, H.B., The Equilibrum Between Creatine and Creatinine in Aqueous Solution. The Effect of Hydrogen Ion. J. Amer. Chem. Soc. 1925, 47, 1179-1188.

Referring now to FIG. 1B, an embodiment of the present invention is shown wherein creatine ester metabolism is shown. By providing a creatine ester, a more water-soluble compound will be provided than the relatively insoluble zwitterionic creatine, and increased lipophilicities will allow for better membrane permeability.

For example, by masking the carboxylic acid functional group of creatinine by esterification, the formation of creatinine in the stomach will be precluded, resulting in an efficient delivery of the creatine esters to the intestine where absorption may occur. Standard supplements containing creatine monohydrate undergo substantial conversion to creatinine in the stomach. This; coupled with the low absorption of creatine in the intestine, leads to reduced amounts of creatine reaching the muscle cell.

In contrast, creatine esters do not undergo conversion to creatinine in the stomach and are more readily absorbed in the intestine. As a result, blood creatine concentrations are higher and thus more creatine is available to the muscle. As a result of this, the intestinal absorption of creatine ester will be significantly greater than that observed with creatine monohydrate. An additional advantage of creatine esters is that, as the creatine ester compound moves from the intestinal tissue into the bloodstream, the creatine ester compounds themselves are biologically inactive, but esterase enzymes present in both the intestinal cells and the blood break the ester bonds of creatine ester, converting it to biologically active creatine. In other words, the advantages of the creatine ester are preserved during transport, such as increased solubility and permeability, but when needed, the creatine is available to be converted into its biologically active form.

Compared to creatine monohydrate, the increased blood levels of creatine obtained with supplements containing the creatine ester compounds are expected to result in increased responses at the target tissue (i.e. muscle). Thus the increased stability and improved absorption of creatine ester results in much greater blood creatine levels than can be achieved with creatine monohydrate supplements. Once in the blood, creatine is transported into the muscle cells, where it is converted to creatine phosphate that will then be consumed by the cell during muscle performance.

Following is a brief overview of the various disease states that may be responsive to creatine supplementation. It should be noted that the proposed disease states below involve increasing creatine in a diverse array of cells including not only muscle but neurons and endothelial cells as well.

### Parkinson's Disease

Parkinson's disease depletes dopamine levels in the brain. Energy impairment may play a role in the loss of dopaminergic neurons. Studies involving rats showed that a diet supplemented with creatine for 2 weeks resulted in only a 10% reduction in brain dopamine as compared to a 70% dopamine depletion in nonsupplemented rodents. *See* Matthews RT, Ferrante RJ, Klivenyi P, Yang L, Klein AM, Mueller G, Kaddurah-Daouk R and Beal MF. Creatine and cyclocreatine attenuate MPTP neurotoxicity. Exp Neurol. 157: 142-149, (1999).

These pre-clinical studies suggest that creatine dietary supplements may have a positive therapeutic outcome in slowing the onset and decreasing the severity of the disease.

### Huntington's Disease

Alterations in energy production may also contribute to the development of brain lesions in patients with Huntington's disease. Rats fed a diet supplemented with creatine for 2 weeks responded better when exposed to 3-nitropropionic acid which mimics the changes in energy metabolism seen with Huntington's disease. The creatine fed animals had 83% less lesion volume than nonsupplemented animals (Matthews et al.,1999).

### Mitochondrial Pathologies

Creatine supplementation increased the life-span of GP3A transgenic mice (a model for amyotrophic lateral sclerosis) up to 26 days. A study involving patients with a variety of neuromuscular disorders also benefited from creatine supplementation, *See* Klivenyi P. Ferrante RJ, Matthews RT, Bogdanov MB, Klein AM, Andreassen OA, Mueller G, Wermer M, Kaddurah-Daouk R and Beal MF. Neuroprotective effects of creatine in a transgenic animal model of amyotrophic lateral sclerosis. Nat Med 5: 347-350, (1999). Increases in high-density strength measurements were seen in these patients following a short-term trail of creatine (10g/d for 5 days with 5g/d for 5 to 7 days). Creatine supplementation also resulted in increased body weight in these patients.

### Stroke

Creatine may also be useful in patients with hypoxia and ishemic brain diseases such as stroke. Creatine has been shown to reduce damage to the brainstem and hippocampus resulting from hypoxia. *See* Balestrino M, Rebaudo R and Lunardi G. Exogenous creatine delays anoxic depolarization and protects from hypoxic damage: Dose-effect relationship. Brain Res 816:124-130, (1999); and Dechent P, Pouwels PJ, Wilken B, Hanefeld F and Frahm J. Increase of total creatine in human brain after oral supplementation of creatine-monohydrate. Am J Physiol 277: R698-R704, (1999). This neuroprotection may be due to prevention of ATP depletion. Studies suggest that supplementation of humans with creatine does increase brain levels of creatine. See Wick M, Fujimori H, Michaelis T and Frahm J. Brain water diffusion in normal and creatine-supplemented rats during transient global ischemia. Magn Reson Med 42: 798-802, (1999); Michaelis T, Wick M, Fujimori H, Matsumura A and Frahm J. Proton MRS of oral creatine supplementation in rats. Cerebral metabolite concentrations and ischemic challenge. NMR Biomed 12: 309-314, (1999); and Malcon C, Kaddurah-Daouk R and Beal M. Neuroprotective effects of creatine administration against NMDA and malonate toxicity. Brain Res 860: 195-198, (2000). High brain creatine levels may offer protection to ischemic brain injury.

### Muscular Diseases

Patients with various muscular dystrophies supplemented with creatine for 8 weeks showed a 3% increase in strength and a 10% improvement in neuromuscular symptom score. Short-term creatine supplementation also improved strength in patients with rheumatoid arthritis, but did not change physical function. *See* Felber S, Skladal D, "Wyss M, Kremser C, Koller A and Sperl W. Oral creatine supplementation in Duchenne muscular dystrophy: A clinical and 31P magnetic resonance spectroscopy study. Neurol Res 22: 145-150 (2000). Patients with McArdles disease showed improvements when given creatine. The improvements included reduced frequency of muscle pain and increased exercise performance and strength. Increases in exercise performance where also seen during ischemic episodes. *See* Willer B, Stucki G, Hoppeler H, Bruhlmann P and Krahenbuhl S. Effects of creatine supplementation on muscle weakness in patients with rheumatoid arthritis. Rheumatology 39: 293-298, (2000).

### Heart Disease

Given the role of creatine phosphate as an immediate and readily accessible source of phosphate for regeneration of ATP, it follows that creatine supplementation may have a favorable impact diseases of the heart. In patients with congestive heart failure creatine supplementation produced an increase in exercise performance as measured by strength and endurance. *See* Gordon A, Hultman E, Kaijser L, Kristjansson S, Rolf CJ, Nyquist O and Sylven C. Creatine supplementation in chronic heart failure increases skeletal muscle creatine phosphat and muscle performanmce. Cardiovasc Res 30: 413-418, (1995).

An additional consideration with ramifications in the management of cardiovascular diseases is the report that creatine supplementation can lower cholesterol and triglyceride levels in humans. *See* Earnest CP, Almada AL and Mitchell TL. High-perfomance capillary electrophoresis-pure creatine monohydrate reduces blood lipids in men and women. Clin Sci (Colch) 91:113-118, (1996).

### Muscle Fatigue Secondary to Aging

Research on adults over 60-years of age suggest that creatine supplementation may delay muscle fatigue, but does not affect body composition or strength (Rawson and Clarkson, 2000). *See* Rawson ES and Clarkson PM. Acute creatine suplementation in older men. Int J Sports Med 21: 71-75, (2000). As with many of the therapeutic implication studies, these preliminary experiments were performed over a short (i.e. less than 30-day) period of time, where the effects of creatine supplementation on muscle mass and strength may not be fully demonstrated. While the effects observed in the elderly were not profound, these initial reports suggest the health benefits to this growing population are promising.

Referring now to FIG. 1C, an exemplary embodiment of the present invention is shown wherein a pronutrient derivative of creatine is created through the modification of an acid moiety by ester bond attachment. Creatine 102 is changed by modifying an acid moiety through ester bond attachment 104. For example, creatine may be converted to creatine ethyl ester 106, which has a formula as follows:

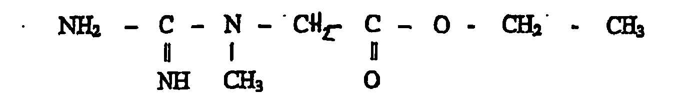

A creatine ester has the advantages of increased aqueous solubility, increased absorption from the gastrointestinal (GI) tract resulting in increased bioavailability, and increased stability, especially for solution formulations. Increased bioavailability allows smaller doses to be utilized with greater effect, thereby resulting in fewer gastrointestinal side effects. Further, more varied formulation possibilities are feasible, for example, the product may be formulated in tablet or capsule form with dextrose and/or phosphate for ease of use and effectiveness.

Once the product is ingested 108, the body metabolizes and activates the product by esterases 110, which may be found in the intestinal lumen, epithelial cells and the blood. The esterases convert the product to creatine 114 and an alcohol 116. Thus, the current invention supplements the amount of creatine normally available to the muscle thereby increasing phosphocreatine levels and decreasing the recovery time required before the muscle can perform activity. Further, the resultant alcohols, such as ethanol, glycerol, benzyl alcohol, tert-butyl alcohol, are relatively harmless. *See* Budavari, S. (Ed.) The Merck Index. Merck and Co., Inc., Whitehouse Station, NJ, 1996. For example, benzyl alcohol is used as a pharmaceutical preservative.

Solubility and permeability are two important factors in the amount of a compound made available to an organism, otherwise known as bioavailability. Solubility refers to the amount of the compound that may be dissolved, wherein permeability refers to the ability of the compound to penetrate across a barrier, such as a membrane, cell wall and the like. In terms of solubility, creatine ethyl ester is a great deal more soluble that creatine. Utilizing a physiological buffer solution (PBS), laboratory analysis indicates that creatine monohydrate has a solubility limit of approximately 10 mg/ml. This value may be overly generous, as a great deal of vortexing of the sample and brief heating of the sample to 37 degrees Celsius had to be performed to even achieve that result. However, the creatine ethyl ester is readily soluble in room temperature PBS with solubility over 200 mg/ml.

With regard to permeability, a laboratory analysis was performed comparing the creatine monohydrate to creatine ethyl ester in MDCK monolayers. The MDCK are a canine kidney epithelial cell line that has been used as an in vitro model for assessing drug permeability. In the MDCK monolayers, creatine monohydrate showed approximately 10% flux over one hour. In other words, 10% of the original amount of creatine monohydrate added to one side of the MDCK monolayer made it across to the other side in a 60-minute period. For creatine ethyl ester, the permeability is quite higher, averaging approximately 20% flux over one hour. Similar results are expected in a Caco-2 monolayer, which may be used as an in vitro model for intestinal absorption. Thus, the creatine ester of the present invention has the unexpected result of both increased solubility and membrane permeability, and thus greater bioavailability, as shown through the following table and graph depicted in FIG. 1D.

| Substance | Conc. at Saturation mg/ml | Partition Coefficient |
|---|---|---|
| Creatine | 15.6+/-2.1 | 0.015 +/- 0.007 |
| Creatine Ethyl Ester | 205.9 +/-1.5 | 0.074 +/- 0.008 |
| Creatine Benzyl Ester | 89.26 +/-0.8 | 0.106 +/-0.01 |

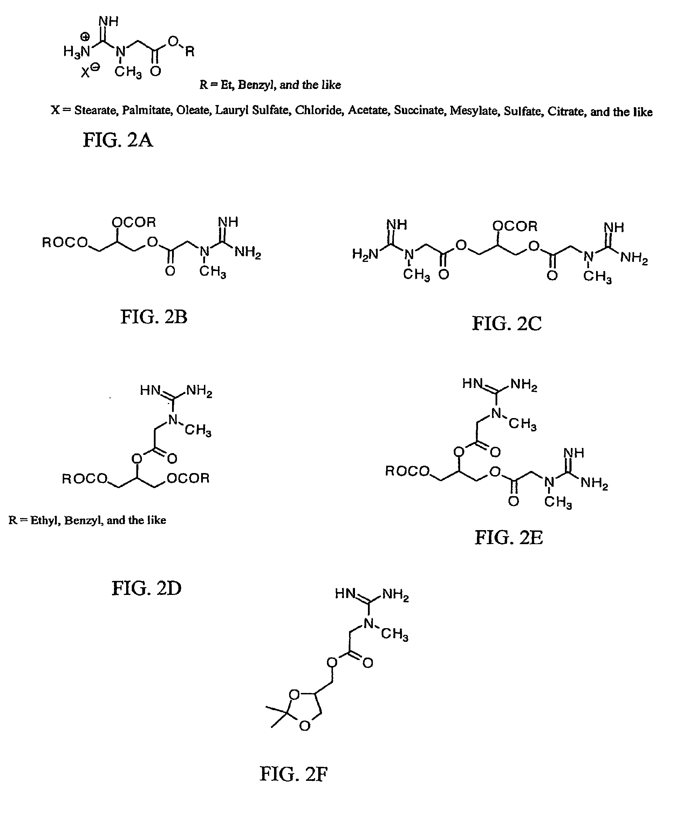

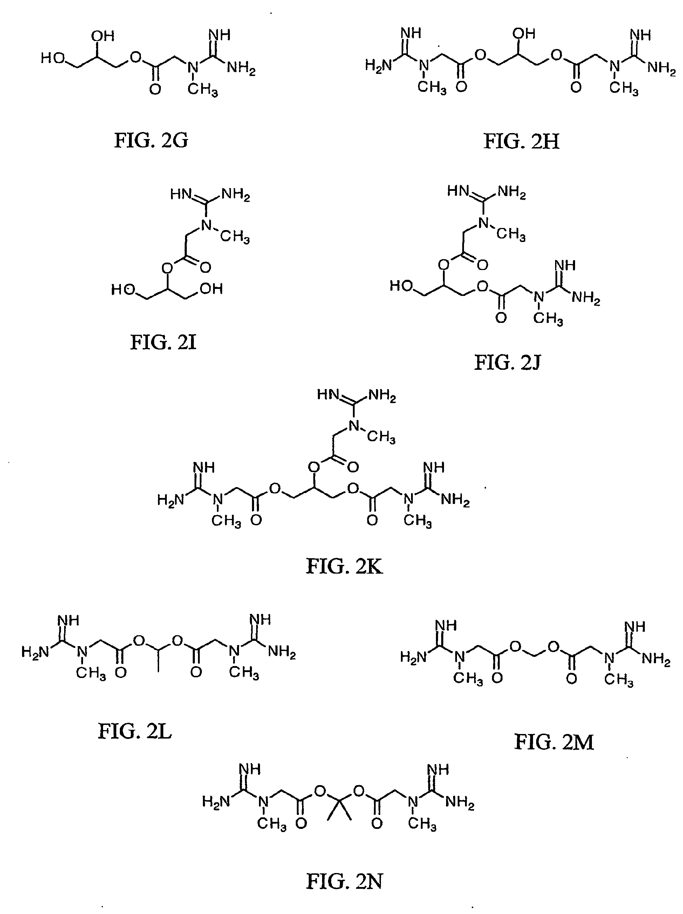

Although a creatine ethyl ester compound has been described, it should be apparent that a wide variety of creatine ester compounds and salts thereof are contemplated by the present invention without departing from the scope thereof, examples of which are shown in FIGS. 2A, 2B, 2C, 2D, 2E, 2F, 2G, 2H, 2I, 2J, 2K, 2L, 2M and 2N. For example, a mono-creatine glycerol, di-creatine glycerol, tricreatine glycerol and the like, may be utilized as a pronutrient of the present invention, the formula for a tricreatine glycerol is as follows:

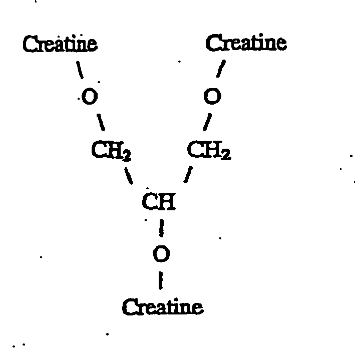

Another example of a creatine ester compound suitable for use as a pronutrient includes creatine phosphoester, the formula of which is as follows: 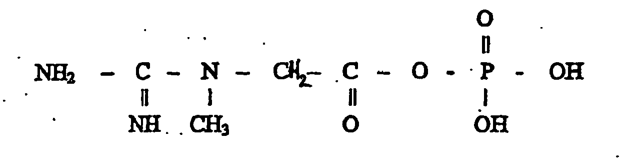 Thus, the present invention provides multiple ester derivatives of creatine for use as pronutrients having increased solubility and permeability over creatine itself. The advantages of creatine pronutrients of the present invention would be useful in athletic performance markets, therapeutic markets targeting patients with diseases involving reduced muscle performance/loss of muscle mass, livestock/animal food products market, and the like.

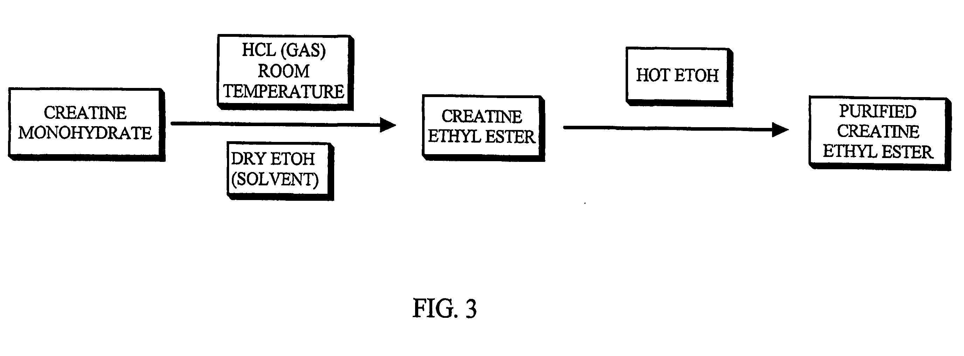

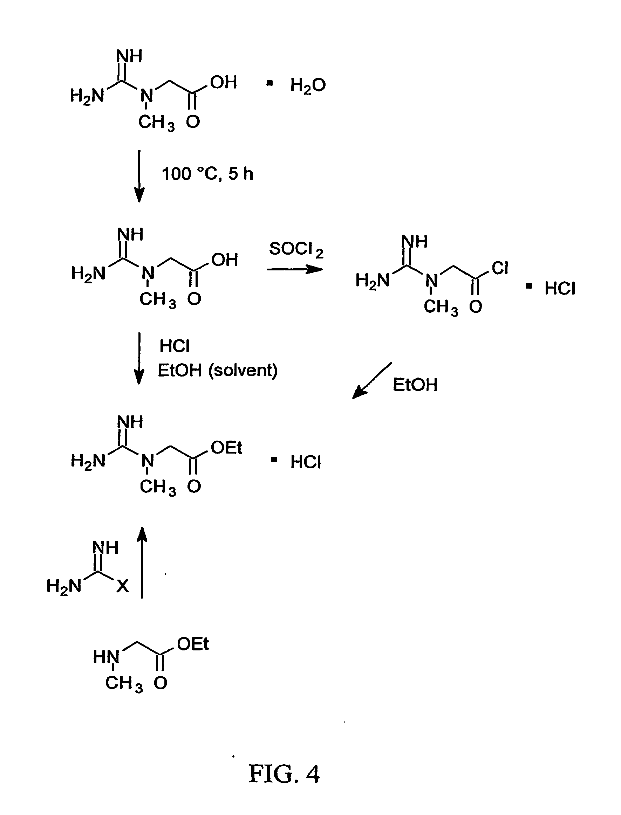

Referring generally now to FIGS. 3 and 4, an exemplary embodiment of the present invention is shown wherein the production of an ester derivative of creatine is shown. A creatine ester may be formed by reacting a hydrated form of creatine or anhydrous creatine with various alcohols in an acidic atmosphere. Under these conditions, various ester procreatine compounds may be formed, generally as white precipitates. The resultant creatine esters may be further purified by solvating in an alcohol at elevated temperatures and then cooling to form the ester procreatine compound. The final recrystallization step may not be required, as the initial precipitate is generally pure. However, such an extra step may be useful to ensure that the purest form of the creatine pronutrient has been obtained.

For example, as shown in FIG. 3, creatine monohydrate may be solvated in dry ethyl alcohol in an atmosphere of hydrochloric acid at ambient temperatures. The resultant creatine ethyl ester compound is solid at ambient temperatures. While not functionally necessary, the resultant creatine ethyl ester may be further purified with the use of ethyl alcohol at an elevated temperature to solvate the creatine ethyl ester away from possible contaminates contained in the solid reaction material. Purified creatine ethyl ester may then be achieved upon cooling the solvated creatine ethyl ester. It should also be apparent that anhydrous creatine may also be utilized.

Although the formulation of creatine ethyl ester is disclosed, it should be apparent that a variety of creatine esters may be produced utilizing analogous reaction systems. *See* Dox., A.W.; Yoder, L Esterification of Creatine. J. Biol. Chem. 1922, 67, 671-673.

For instance, a variety of methods of producing a creatine ester are contemplated without departing from the scope of the present invention, such as the methods and process shown in FIG. 4, wherein X may include a leaving group. Although the use of creatine monohydrate is disclosed, a variety of creatine containing starting compounds are contemplated by the present invention, creatine monohydrate being disclosed merely because of its availability.

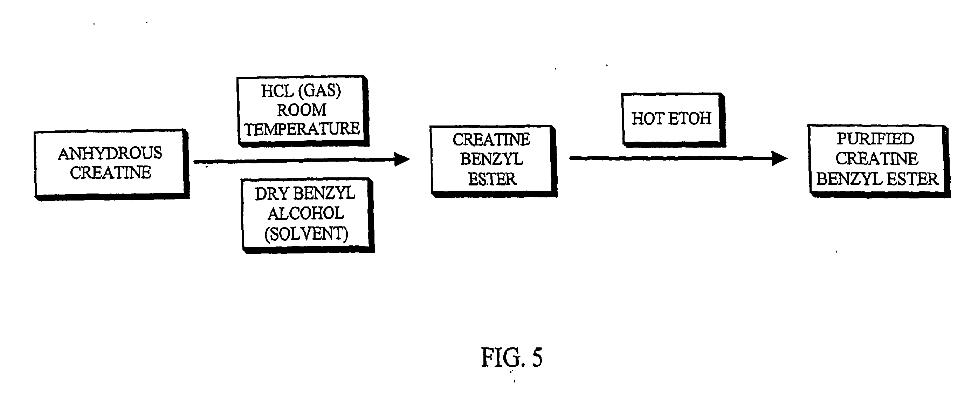

Referring now to FIG. 5, an embodiment of the present invention is shown wherein anhydrous creatine is solvated in dry benzyl alcohol in an atmosphere of hydrochloric acid at ambient temperatures to produce a creatine ester. The resultant creatine benzyl ester compound is a white solid at ambient temperatures. While not functionally necessary, the resultant creatine benzyl ester may be further purified with the use of ethyl alcohol at an elevated temperature to solvate the creatine benzyl ester away from possible contaminates. Purified creatine benzyl ester may then be achieved upon cooling the solvated creatine benzyl ester. As stated earlier, the final recrystallization step may not be required as the initial precipitate in relatively pure. However, such an extra purification step may be useful to ensure that the most pure form of the compound has been obtained.

As discussed earlier, creatine esters may also be synthesized from anhydrous creatine using esterification methods and isolated as their hydrochloride salts. For example, creatine ethyl ester hydrochloride may be synthesized by treatment of anhydrous creatine with ethanolic HCl at room temperature. *See* Dox., A.W.; Yoder, L. Esterification of Creatine. J. Biol. Chem., 67, 671-673, (1922). 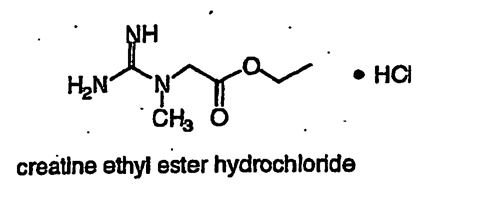 Using this method, creatine ethyl ester hydrochloride was synthesized in 74% yield after a single recrystallization from ethanol.

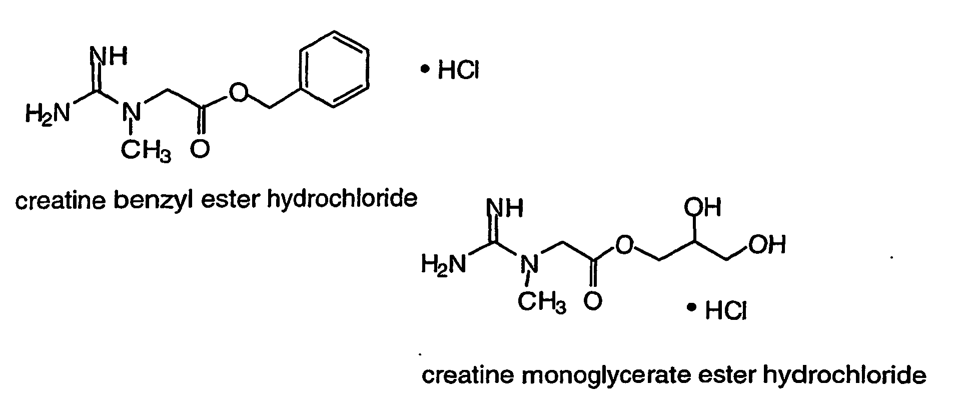

Creatine esters creatine benzyl ester hydrochloride and creatine monoglycerate ester hydrochloride may similarly be obtained by exposure of anhydrous creatine with excess HCl-saturated benzyl alcohol and glycerol, respectively. It should be apparent that stereoisomers, such as a stereoisomers of creatine monoglycerate ester hydrochloride, and the compounds shown in FIGS. 2B, 2E, 2F, 2G, 2J and the like, are also contemplated by the present invention.

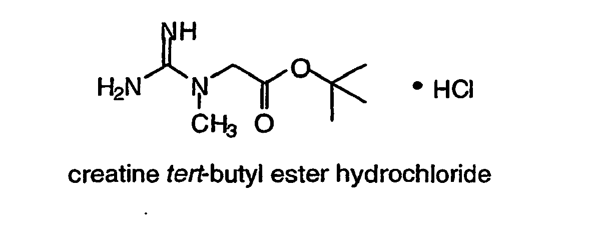

Creatine tert-butyl ester hydrochloride may be obtained by treatment of creatine acid chloride with *tert*-butanol and zinc chloride. *See* Rak, J.; Lubkowski, J.; Nikel, I.; Przubulski, J.; Blazejowski, J. Thermal Properties, Crystal Lattice Energy, Mechanism and Energetics of the Thermal Decomposition of Hydrochlorides of 2-Amino Acid Esters, Thermochimica Acta 171, 253-277 (1990); Yadav, J.S.; Reddy, G.S.; Srinivas, D.; Himabindu, K. Zinc Promoted Mild and Efficient Method for the Esterification of Acid Chlorides with Alcohols, Synthetic Comm. 28, 2337-2342 (1998). Creatine tert-butyl ester hydrochloride may also be obtained by treatment of anhydrous creatine with *tert*-butanol and anhydrous magnesium sulfate and catalytic sulfuric acid. *See* Wright, S.W.; Hageman, D.L; Wright, A.S.; McClure, L.D. Convenient Preparations of t-Butyl Esters and Ethers from t-Butanol, Tetrahedron Lett. 38, 7345-7348 (1997).

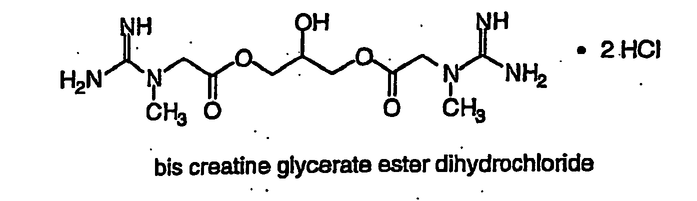

Bis creatine glycerate ester dihydrochloride ester, may be obtained by treatment of creatine acid chloride with a half-molar equivalent of anhydrous glycerol. See Rak, J.; Lubkowski, J.; Nikel, L; Przubulski, J.; Blazejowski, J. Thermal Properties. Crystal Lattice Energy, Mechanism and Energetics of the Thermal. Decomposition of Hydrochlorides of 2-Amino Acid Ester, Thermochimica Acta 71, 253-277 (1990).

Alternatives to these methods include transesterification reaction of CE1 using either catalytic diphenyl ammonium triflate and trimethylsilyl chloride (Wakasugi et al., 2000) or catalytic potassium *tert-butoxide* and 1 equivalent of *tert-*butyl acetate. Creatine acid chloride may also be used rather than anhydrous creatine in the esterification reactions. *See* Wakasugi, K.; Misake, T.; Yamada, K.; Tanabe. Y Diphenylammonium triflate (DPAT): Efficient Catalyst for Esterification of Carboxylic Acids and For Transesterification of Carboxylic Esters With Nearly Equimolar Amounts of Alcohols, Tetrahedron Lett 41, 5249-5252 (2000).

Regioselectivity problems in the formation of creatine esters, such as creatine monoglycerate ester hydrochloride, Bis creatine glycerate ester dihydrochloride ester, and the like, may be addressed by selective esterification of the primary alcohol functional group(s) of glycerol with creatine acid chloride in the presence of *N,N-*diisopropylethylamine or 2,4,6-collidine at low temperatures. *See* Ishihara, K.; Kurihara, H; Yamamoto, H. An Extremely Simple, Convenient, and Selective Method for Acetylating Primary Alcohols in the Presence of Secondary Alcohols, J. Org Chem. 58, 3791-3793 (1993).

Creatine esters may be purified by crystallization, flash column chromatography, and the like, if desired, and the structures and purity confirmed by analytical HPLC. ¹H and ¹³C NMR, IR, melting point and elemental analysis. The following data was obtained through nuclear magnetic resonance spectroscopy of the corresponding compounds:
Creatine ethyl ester hydrochloride
   ¹H NMR (500 MHz, CDCl₃) δ 1.12 (dq, J = 6.0 Hz, J =1.0 Hz, 3H), 2.91, (s, 3H), 4.10-4.11 (m, 4H).
Creatine benzyl ester hydrochloride
   ¹H NMR (500 MHz, DMSO-d₆) δ 3.03 (s, 3H), 4.13 (s, 2H), 5.06 (s, 2H), 7.22-7.38 (m, 5H).

It is understood that the specific order or hierarchy of steps in the methods disclosed are examples of exemplary approaches. Based upon design preferences, it is understood that the specific order or hierarchy of steps in the method can be rearranged while remaining within the scope of the present invention. The accompanying method claims present elements of the various steps in a sample order, and are not meant to be limited to the specific order or hierarchy presented.

It is believed that the creatine ester pronutrient compounds and formulations of the present invention and many of its attendant advantages will be understood by the forgoing description.

## Claims

1. A non-therapeutic method for providing creatine to an animal, comprising:
receiving a creatine ester by the animal, wherein the creatine ester is suitable for being modified by the animal to form creatine, and wherein the creatine ester includes at least one of creatine ethyl ester, creatine benzyl ester, creatine phosphoester, monocreatine glycerol, t-butyl creatine ester, dicreatine glycerol and tricreatine glycerol.

2. A method according to claim 1 wherein the creatine ester is creatine ethyl ester.

3. A method according to claim 1 or 2, wherein the creatine ester is suitable for being formed in a solid form capable of being ingested by the animal.

4. A method according to claim 3, wherein the solid form includes the creatine ester and at least one of dextrose and phosphate.

5. A method according to claim 3, wherein the solid form is configured as at least one of a tablet and a capsule.

6. A method according to claim 1 or 2, wherein the creatine ester is suitable for liquid delivery.

7. A method according to claim 6, wherein the creatine ester includes at least one of an aqueous solution and emulsion.

8. A method according to any preceding claim, wherein the creatine ester is received by the animal, the creatine ester is modified by the animal into creatine and an alcohol.

9. A method according to claim 8, wherein the creatine ester is modified by the animal into creatine and alcohol by an esterase.

10. A method according to claim 8, wherein the creatine ester is modified by at least one of an intestinal lumen, epithelial cell and blood of the animal into creatine.

11. A method according to any preceding claim, further comprising forming the creatine ester, wherein an acid moiety of creatine is modified to provide an ester bond.

12. A method according to any preceding claim, wherein the animal is human.

13. A method according to any of claims 1 to 11 wherein the animal is livestock.

14. A method according to any preceding claim, wherein the creatine ester is suitable for acting as a pronutrient in an animal.

15. A method according to claim 14, wherein the creatine ester acts as a pronutrient in the gastrointestinal tract of the animal.

16. A method according to claim 14, wherein the pronutrient is metabolized by the animal to form creatine.

17. A method according to claim 16, wherein the pronutrient is metabolized by an esterase.

18. A method according to claim 17, wherein the pronutrient is metabolized by esterases in at least one of an intestinal lumen, epithelial call and blood.

19. A method according to claim 14, wherein the pronutrient is metabolized by the animal to form an alcohol.

20. A food supplement, comprising:
a creatine ester suitable for being modified by an animal to form creatine, wherein the creatine ester includes at least one of creatine ethyl ester, creatine benzyl ester, creatine phosphoester, monocreatine glycerol, t-butyl creatine ester, dicreatine glycerol and tricreatine glycerol.

21. A food supplement according to claim 20, wherein the creatine ester is creatine ethyl ester.

22. A food supplement according to claim 20 or 21, wherein the creatine ester is suitable for being formed in a solid form capable of being ingested by the animal.

23. A food supplement according to claim 22, wherein the solid form includes the creatine ester and at least one of dextrose and phosphate.

24. A food supplement according to claim 22, wherein the solid form is configured as at least one of a tablet and a capsule.

25. A food supplement according to claim 20 or 21, wherein the creatine ester is suitable for liquid delivery.

26. A food supplement according to claim 25, wherein the creatine ester includes at least one of an aqueous solution and emulsion.

27. A food supplement according to any of claims 20 to 26, wherein the creatine ester is received by the animal, the creatine ester is modified by the animal into creatine and an alcohol

28. A food supplement according to claim 27, wherein the creatine ester is modified by the animal into creatine and alcohol by an esterase.

29. A food supplement according to claim 27, wherein the creatine ester is modified by at least one of an intestinal lumen, epithelial cell and blood of the animal into creatine.

30. A food supplement according to any of claims 20 to 29, further comprising forming the creatine ester, wherein an acid moiety of creatine is modified to provide an ester bond.

31. A food supplement according to any of claims 20 to 30 wherein the animal is human.

32. A food supplement according to any of claims 20 to 30, wherein the animal is livestock.

33. A food supplement according to any of claims 20 to 32, wherein the creatine ester has the structure: 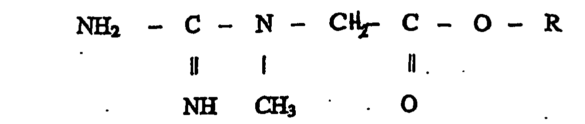 wherein R represents an ester.

34. A food supplement according to claim 33, wherein the creatine ester comprises a salt.

35. A method of producing a creatine pronutrient, comprising:
reacting at least one of an anhydrous creatine and hydrated form of creatine with an alcohol in an acidic environment, wherein a product is formed including a creatine ester pronutrient.

36. A method according to claim 35, wherein the hydrated form of creatine includes creatine monohydrate.

37. A method according to claim 35, wherein the alcohol includes at least one of ethyl alcohol and benzyl alcohol.

38. A method according to claim 35, wherein the acidic environment is achieved through hydrochloric acid being present.

39. A method according to claim 35, further comprising purifying the product.

40. A method according to claim 39, wherein the product is purified by solvating the product in an alcohol and then cooling to form purified creatine ester.

41. A method according to any of claims 35 to 40,
wherein the creatine pronutrient is creatine ethyl ester.

42. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of Parkinson's disease.

43. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of Huntington's disease.

44. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of mitochondrial pathologies.

45. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of stroke.

46. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of muscular diseases.

47. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of heart disease.

48. Use of a creatine ester which includes at least one of creatine ethyl ester, creatine benzyl ester, creatine phosphoester, monocreatine glycerol, t-butyl creatine ester, dicreatine glycerol and tricreatine glycerol, in the preparation of a medicament for the prevention and/or treatment of muscle fatigue secondary to aging.

49. A use according to any of Claims 42 to 48, wherein the creatine ester is creatine ethyl ester.

50. A use according to any of Claims 42 to 49, wherein the creatine ester is suitable for being formed in a solid form capable of being ingested by an animal.

51. A use according to Claim 50, wherein the solid form includes the creatine ester and at least one of dextrose and phosphate.

52. A use according to Claim 50, wherein the solid form is configured as at least one of a tablet and a capsule.

53. A use according to any of Claims 42 to 49, wherein the creatine ester is suitable for liquid delivery.

54. A use according to Claim 53, wherein the creatine ester includes at least one of an aqueous solution and emulsion.

55. A use according to any of Claims 42 to 54, wherein the creatine ester is suitable to be modified by the animal into creatine and an alcohol.

56. A use according to Claim 55, wherein the creatine ester is suitable to be modified by the animal into creatine and alcohol by an esterase.

57. A use according to Claim 55, wherein the creatine ester is suitable to be modified by at least one of an intestinal lumen, epithelial cell and blood of the animal into creatine.

58. A use according to any of Claims 42 to 54, further comprising forming a creatine ester, wherein an acid moiety of creatine is modified to provide an ester bond.

59. A use according to any of Claims 42 to 58, wherein the animal is human.

60. A use according to any of Claims 42 to 58 wherein the animal is livestock.

## Patentansprüche

1. Nichttherapeutisches Verfahren zur Versorgung eines tierischen Lebewesens mit Kreatin, das das Aufnehmen eines Kreatinesters durch das tierische Lebewesen umfasst, wobei der Kreatinester zur Modifikation durch das tierische Lebewesen unter Bildung von Kreatin geeignet ist und wobei der Kreatinester mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst.

2. Verfahren nach Anspruch 1, wobei der Kreatinester Kreatinethylester ist.

3. Verfahren nach Anspruch 1 oder 2, wobei der Kreatinester in einer festen Form, die von dem tierischen Lebewesen eingenommen werden kann, ausgebildet werden kann.

4. Verfahren nach Anspruch 3, wobei die feste Form den Kreatinester und Dextrose und/oder ein Phosphat umfasst.

5. Verfahren nach Anspruch 3, wobei die feste Form als Tablette und/oder Kapsel konfiguriert ist.

6. Verfahren nach Anspruch 1 oder 2, wobei der Kreatinester zur flüssigen Zufuhr geeignet ist.

7. Verfahren nach Anspruch 6, wobei der Kreatinester eine wässrige Lösung und/oder Emulsion umfasst.

8. Verfahren nach einem der vorhergehenden Ansprüche, wobei der Kreatinester von dem tierischen Lebewesen aufgenommen wird, der Kreatinester durch das tierische Lebewesen zu Kreatin und einem Alkohol modifiziert wird.

9. Verfahren nach Anspruch 8, wobei der Kreatinester durch das tierische Lebewesen zu Kreatin und einem Alkohol durch eine Esterase modifiziert wird.

10. Verfahren nach Anspruch 8, wobei der Kreatinester durch das Darmlumen und/oder Epithelzellen und/oder Blut des tierischen Lebewesens zu Kreatin modifiziert wird.

11. Verfahren nach einem der vorhergehenden Ansprüche, das ferner die Bildung des Kreatinesters, wobei eine Säureeinheit von Kreatin unter Bildung einer Esterbindung modifiziert wird, umfasst.

12. Verfahren nach einem der vorhergehenden Ansprüche, wobei das tierische Lebewesen ein Mensch ist.

13. Verfahren nach einem der Ansprüche 1 bis 11, wobei das tierische Lebewesen ein Nutztier ist.

14. Verfahren nach einem der vorhergehenden Ansprüche, wobei der Kreatinester geeignet ist, als Pronährstoff in einem tierischen Lebewesen zu wirken.

15. Verfahren nach Anspruch 14, wobei der Kreatinester als Pronährstoff im Gastrointestinaltrakt des tierischen Lebewesens wirkt.

16. Verfahren nach Anspruch 14, wobei der Pronährstoff durch das tierische Lebewesen unter Bildung von Kreatin metabolisiert wird.

17. Verfahren nach Anspruch 16, wobei der Pronährstoff durch eine Esterase metabolisiert wird.

18. Verfahren nach Anspruch 17, wobei der Pronährstoff durch Esterasen in dem Darmlumen und/oder Epithelzellen und/oder Blut metabolisiert wird.

19. Verfahren nach Anspruch 14, wobei der Pronährstoff durch das tierische Lebewesen unter Bildung eines Alkohols metabolisiert wird.

20. Nahrungsergänzungsmittel, das einen Kreatinester umfasst, der zur Modifikation durch ein tierisches Lebewesen unter Bildung von Kreatin geeignet ist, wobei der Kreatinester mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst.

21. Nahrungsergänzungsmittel nach Anspruch 20, wobei der Kreatinester Kreatinethylester ist.

22. Nahrungsergänzungsmittel nach Anspruch 20 oder 21, wobei der Kreatinester in einer festen Form, die von dem tierischen Lebewesen eingenommen werden kann, ausgebildet werden kann.

23. Nahrungsergänzungsmittel nach Anspruch 22, wobei die feste Form den Kreatinester und Dextrose und/oder ein Phosphat umfasst.

24. Nahrungsergänzungsmittel nach Anspruch 22, wobei die feste Form als Tablette und/oder Kapsel konfiguriert ist.

25. Nahrungsergänzungsmittel nach Anspruch 20 oder 21, wobei der Kreatinester zur flüssigen Zufuhr geeignet ist.

26. Nahrungsergänzungsmittel nach Anspruch 25, wobei der Kreatinester eine wässrige Lösung und/oder Emulsion umfasst.

27. Nahrungsergänzungsmittel nach einem der Ansprüche 20 bis 26, wobei der Kreatinester von dem tierischen Lebewesen aufgenommen wird, der Kreatinester durch das tierische Lebewesen zu Kreatin und einem Alkohol modifiziert wird.

28. Nahrungsergänzungsmittel nach Anspruch 27, wobei der Kreatinester durch das tierische Lebewesen zu Kreatin und einem Alkohol durch eine Esterase modifiziert wird.

29. Nahrungsergänzungsmittel nach Anspruch 27, wobei der Kreatinester durch das Darmlumen und/oder Epithelzellen und/oder Blut des tierischen Lebewesens zu Kreatin modifiziert wird.

30. Nahrungsergänzungsmittel nach einem der Ansprüche 20 bis 29, das ferner die Bildung des Kreatinesters, wobei eine Säureeinheit von Kreatin unter Bildung einer Esterbindung modifiziert wird, umfasst.

31. Nahrungsergänzungsmittel nach einem der Ansprüche 20 bis 30, wobei das tierische Lebewesen ein Mensch ist.

32. Nahrungsergänzungsmittel nach einem der Ansprüche 20 bis 30, wobei das tierische Lebewesen ein Nutztier ist.

33. Nahrungsergänzungsmittel nach einem der Ansprüche 20 bis 32, wobei der Kreatinester die folgende Struktur aufweist: 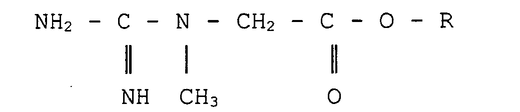 worin R für einen Ester steht.

34. Nahrungsergänzungsmittel nach Anspruch 33, wobei der Kreatinester ein Salz umfasst.

35. Verfahren zur Herstellung eines Kreatin-Pronährstoffs, das die Umsetzung von wasserfreiem Kreatin und/oder einer Hydratform von Kreatin mit einem Alkohol in einer sauren Umgebung umfasst, wobei ein Produkt gebildet wird, das einen Kreatinester-Pronährstoff umfasst.

36. Verfahren nach Anspruch 35, wobei die Hydratform von Kreatin Kreatinmonohydrat umfasst.

37. Verfahren nach Anspruch 35, wobei der Alkohol Ethylalkohol und/oder Benzylalkohol umfasst.

38. Verfahren nach Anspruch 35, wobei die saure Umgebung **dadurch** erreicht wird, dass Salzsäure vorhanden ist.

39. Verfahren nach Anspruch 35, das ferner eine Reinigung des Produkts umfasst.

40. Verfahren nach Anspruch 39, wobei das Produkt durch Solvatisieren des Produkts in einem Alkohol und dann Kühlen gereinigt wird, wobei gereinigter Kreatinester gebildet wird.

41. Verfahren nach einem der Ansprüche 35 bis 40, wobei der Kreatin-Pronährstoff Kreatinethylester ist.

42. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung von Parkinson-Krankheit.

43. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung von Chorea Huntington.

44. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung mitochondrialer Pathologien.

45. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung eines Schlaganfalls.

46. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung von Muskelerkrankungen.

47. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung einer Herzkrankheit.

48. Verwendung eines Kreatinesters, der mindestens eine Spezies von Kreatinethylester, Kreatinbenzylester, Kreatinphosphoester, Monokreatinglycerin, Kreatin-tert-butylester, Dikreatinglycerin und Trikreatinglycerin umfasst, bei der Herstellung eines Medikaments zur Prävention und/oder Behandlung von altersbedingter Muskelschwäche.

49. Verwendung nach einem der Ansprüche 42 bis 48, wobei der Kreatinester Kreatinethylester ist.

50. Verwendung nach einem der Ansprüche 42 bis 49, wobei der Kreatinester in einer festen Form, die von dem tierischen Lebewesen eingenommen werden kann, ausgebildet werden kann.

51. Verwendung nach Anspruch 50, wobei die feste Form den Kreatinester und Dextrose und/oder ein Phosphat umfasst.

52. Verwendung nach Anspruch 50, wobei die feste Form als Tablette und/oder Kapsel konfiguriert ist.

53. Verwendung nach einem der Ansprüche 42 bis 49, wobei der Kreatinester zur flüssigen Zufuhr geeignet ist.

54. Verwendung nach Anspruch 53, wobei der Kreatinester eine wässrige Lösung und/oder Emulsion umfasst.

55. Verwendung nach einem der Ansprüche 42 bis 54, wobei der Kreatinester zur Modifikation durch das tierische Lebewesen zu Kreatin und einem Alkohol geeignet ist.

56. Verwendung nach Anspruch 55, wobei der Kreatinester zur Modifikation durch das tierische Lebewesen zu Kreatin und einem Alkohol durch eine Esterase geeignet ist.

57. Verwendung nach Anspruch 55, wobei der Kreatinester zur Modifikation durch das Darmlumen und/oder Epithelzellen und/oder Blut des tierischen Lebewesens zu Kreatin geeignet ist.

58. Verwendung nach einem der Ansprüche 42 bis 54, die ferner die Bildung des Kreatinesters, wobei eine Säureeinheit von Kreatin unter Bildung einer Esterbindung modifiziert wird, umfasst.

59. Verwendung nach einem der Ansprüche 42 bis 58, wobei das tierische Lebewesen ein Mensch ist.

60. Verwendung nach einem der Ansprüche 42 bis 58, wobei das tierische Lebewesen ein Nutztier ist.

## Revendications

1. Procédé non thérapeutique destiné à fournir de la créatine à un animal, comprenant l'étape consistant à :
recevoir un ester de créatine par l'animal, dans lequel l'ester de créatine est approprié pour être modifié par l'animal pour former de la créatine, et dans lequel l'ester de créatine comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol.

2. Procédé selon la revendication 1, dans lequel l'ester de créatine est l'ester d'éthyle de créatine.

3. Procédé selon la revendication 1 ou 2, dans lequel l'ester de créatine est approprié pour être formé sous une forme solide capable d'être ingérée par l'animal.

4. Procédé selon la revendication 3, dans lequel la forme solide comprend l'ester de créatine et au moins l'un parmi le dextrose et un phosphate.

5. Procédé selon la revendication 3, dans lequel la forme solide est configurée en au moins un parmi un comprimé et une capsule.

6. Procédé selon la revendication 1 ou 2, dans lequel l'ester de créatine est approprié pour une administration liquide.

7. Procédé selon la revendication 6, dans lequel l'ester de créatine comprend au moins l'un parmi une solution aqueuse et une émulsion.

8. Procédé selon l'une quelconque des revendications précédentes, dans lequel l'ester de créatine est reçu par l'animal, l'ester de créatine est modifié par l'animal en créatine et un alcool.

9. Procédé selon la revendication 8, dans lequel l'ester de créatine est modifié par l'animal en créatine et en alcool par une estérase.

10. Procédé selon la revendication 8, dans lequel l'ester de créatine est modifié en créatine par au moins l'un parmi la lumière intestinale, une cellule épithéliale et le sang de l'animal.

11. Procédé selon l'une quelconque des revendications précédentes, comprenant en outre l'étape consistant à former l'ester de créatine, dans laquelle une entité acide de créatine est modifiée pour former une liaison ester.

12. Procédé selon l'une quelconque des revendications précédentes, dans lequel l'animal est un être humain.

13. Procédé selon l'une quelconque des revendications 1 à 11, dans lequel l'animal est du bétail.

14. Procédé selon l'une quelconque des revendications précédentes, dans lequel l'ester de créatine est approprié pour agir comme pronutriment chez un animal.

15. Procédé selon la revendication 14, dans lequel l'ester de créatine agit comme un pronutriment dans le tractus gastro-intestinal de l'animal.

16. Procédé selon la revendication 14, dans lequel le pronutriment est métabolisé par l'animal pour former de la créatine.

17. Procédé selon la revendication 16, dans lequel le , pronutriment est métabolisé par une estérase.

18. Procédé selon la revendication 17, dans lequel le pronutriment est métabolisé par des estérases dans au moins l'un parmi la lumière intestinale, une cellule épithéliale et le sang.

19. Procédé selon la revendication 14, dans lequel le pronutriment est métabolisé par l'animal pour former un alcool.

20. Complément alimentaire, comprenant :
un ester de créatine approprié pour être modifié par un animal pour former de la créatine, dans lequel l'ester de créatine comprend l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol.

21. Complément alimentaire selon la revendication 20, dans lequel l'ester de créatine est l'ester d'éthyle de créatine.

22. Complément alimentaire selon la revendication 20 ou 21, dans lequel l'ester de créatine est approprié pour être formé sous une forme solide capable d'être ingérée par l'animal.

23. Complément alimentaire selon la revendication 22, dans lequel la forme solide comprend l'ester de créatine et au moins l'un parmi le dextrose et un phosphate.

24. Complément alimentaire selon la revendication 22, dans lequel la forme solide est configurée en au moins un parmi un comprimé et une capsule.

25. Complément alimentaire selon la revendication 20 ou 22, dans lequel l'ester de créatine est approprié pour une administration liquide.

26. Complément alimentaire selon la revendication 25, dans lequel l'ester de créatine comprend au moins l'un parmi une solution aqueuse et une émulsion.

27. Complément alimentaire selon l'une quelconque des revendications 20 à 26, dans lequel l'ester de créatine est reçu par l'animal, l'ester de créatine est modifié par l'animal en créatine et un alcool.

28. Complément alimentaire selon la revendication 27, dans lequel l'ester de créatine est modifié par l'animal en créatine et en alcool par une estérase.

29. Complément alimentaire selon la revendication 27, dans lequel l'ester de créatine est modifié en créatine par au moins l'un parmi la lumière intestinale, une cellule épithéliale et le sang de l'animal.

30. Complément alimentaire selon l'une quelconque des revendications 20 à 29, comprenant en outre l'étape consistant à former l'ester de créatine, dans laquelle une entité acide de créatine est modifiée pour former une liaison ester.

31. Complément alimentaire selon l'une quelconque des revendications 20 à 30, dans lequel l'animal est un être humain.

32. Complément alimentaire selon l'une quelconque des revendications 20 à 30, dans lequel l'animal est du bétail.

33. Complément alimentaire selon l'une quelconque des revendications 20 à 32, dans lequel l'ester de créatine répond à la structure . 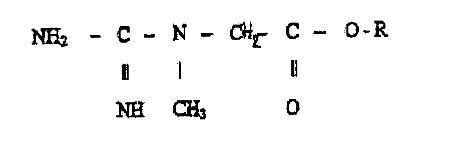 où R représente un ester.

34. Complément alimentaire selon la revendication 33, dans lequel l'ester de créatine comprend un sel.

35. Procédé de production d'un pronutriment à base de créatine, comprenant l'étape consistant à :
faire réagir au moins l'un parmi une créatine anhydre et une forme hydratée de créatine avec un alcool dans un environnement acide, dans lequel un produit est formé comprenant un pronutriment à base d'ester de créatine.

36. Procédé selon la revendication 35, dans lequel la forme hydratée de la créatine comprend le monohydrate de créatine.

37. Procédé selon la revendication 35, dans lequel l'alcool comprend au moins l'un parmi l'alcool éthylique et l'alcool benzylique.

38. Procédé selon la revendication 35, dans lequel l'environnement acide est obtenu par la présence d'acide chlorhydrique.

39. Procédé selon la revendication 35, comprenant en outre la purification du produit.

40. Procédé selon la revendication 39, dans lequel le produit est purifié en solvatant le produit dans un alcool, puis en refroidissant pour produire de l'ester de créatine purifié.

41. Procédé selon l'une quelconque des revendications 35 à 40, dans lequel le pronutriment à base de créatine est l'ester d'éthyle de créatine.

42. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement de la maladie de Parkinson.

43. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement de la maladie de Huntington.

44. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement des pathologies mitochondriales.

45. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement d'un accident cardiovasculaire.

46. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement des maladies musculaires.

47. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement d'une cardiopathie.

48. Utilisation d'un ester de créatine qui comprend au moins l'un parmi l'ester d'éthyle de créatine, l'ester de benzyle de créatine, le phosphoester de créatine, le monocréatine glycérol, l'ester de t-butyle de créatine, le dicréatine glycérol et le tricréatine glycérol, pour la préparation d'un médicament destiné à la prévention et/ou au traitement d'une fatigue musculaire secondaire au vieillissement.

49. Utilisation selon l'une quelconque des revendications 42 à 48, dans laquelle l'ester de créatine est l'ester d'éthyle de créatine.

50. Utilisation selon l'une quelconque des revendications 42 à 49, dans laquelle l'ester de créatine est approprié pour être formé sous une forme solide capable d'être ingérée par un animal.

51. Utilisation selon la revendication 50, dans laquelle la forme solide comprend l'ester de créatine et au moins l'un parmi le dextrose et un phosphate.

52. Utilisation selon la revendication 50, dans laquelle la forme solide est configurée en au moins un parmi un comprimé et une capsule.

53. Utilisation selon l'une quelconque des revendications 42 à 49, dans laquelle l'ester de créatine est approprié pour une administration liquide.

54. Utilisation selon la revendication 53, dans laquelle l'ester de créatine comprend au moins l'un parmi une solution aqueuse et une émulsion.

55. Utilisation selon l'une quelconque des revendications 42 à 54, dans laquelle l'ester de créatine est approprié pour être modifié par l'animal en créatine et un alcool.

56. Utilisation selon la revendication 55, dans laquelle l'ester de créatine est modifié par l'animal en créatine et en alcool par une estérase.

57. Utilisation selon la revendication 55, dans laquelle l'ester de créatine est approprié pour être modifié en créatine par au moins l'un parmi la lumière intestinale, une cellule épithéliale et le sang de l'animal.

58. Utilisation selon l'une quelconque des revendications 42 à 54, comprenant en outre l'étape consistant à former l'ester de créatine, dans laquelle une entité acide de créatine est modifiée pour former une liaison ester.

59. Utilisation selon l'une quelconque des revendications 42 à 58, dans laquelle l'animal est un être humain.

60. Utilisation selon l'une quelconque des revendications 42 à 58, dans laquelle l'animal est du bétail.
